# EUROPEAN PATENT SPECIFICATION

(11) **EP 2 767 339 B1**
(45) Date of publication and mention of the grant of the patent: **11.05.2016**
(21) Application number: 14152749.9
(22) Date of filing: 27.01.2014
(51) Int. Cl.: B01L 3/00

(54) **FIRST WASTE COMPARTMENT WITH CAPILLARY GROOVE FOR ISOBARIC WASTE ENTRY COUPLED TO SECOND WASTE COMPARTMENT**
ERSTE ABFALLKAMMER MIT KAPILLARNUT FÜR ISOBARISCHEN ABFALLEINTRAG MIT ZWEITER ABFALLKAMMER GEKOPPELT
PREMIÈRE CHAMBRE DE DÉCHETS AVEC RAINURE CAPILLAIRE POUR ENTRÉE ISOBARE DE DÉCHETS COUPLÉE À DEUXIÈME CHAMBRE DE DECHÉTS

(30) Priority: 15.02.2013 US 201313768320
(43) Date of publication of application: 20.08.2014
(73) Proprietor: Honeywell International Inc., Morris Plains, NJ 07950 (US)
(72) Inventor: Peltola, Eric Richard, Morristown, NJ New Jersey 07962-2245 (US); Bardell, Ronald, Morristown, NJ New Jersey 07962-2245 (US); Washa, Mark, Morristown, NJ New Jersey 07962-2245 (US)
(74) Representative: Houghton, Mark Phillip

(56) References cited:
- EP-A2- 1 566 215
- WO-A1-2009/137244
- WO-A2-2006/115663
- WO-A2-2008/014050
- US-A1- 2003 152 994
- US-A1- 2007 025 875
- US-A1- 2009 181 411
- US-A1- 2009 215 125

## Description

### Background

Detection of biological samples results in biological waste being generated. There are significant safety issues involved in properly handling and disposing of biological waste. When using disposable cards to distribute and test the waste, the cards should control the waste well enough that instruments that come in contact with the card are not contaminated. Waste chambers have been used to help control the waste.

### Summary

The present invention provides a multilayer cytometric test card and method as defined in the appended claims.

A multiple layer cytometric test card as described in claim 1 includes a waste channel to receive biological waste from an area of the test card utilized for testing biological samples, a first waste storage compartment in a waste layer of the card having a top and a bottom in relation to an operating vertical orientation of the test card, and a capillary positioned along a vertical length of the first waste storage compartment, the capillary being open to the waste compartment along the vertical length of the waste compartment, wherein the waste channel is coupled to the capillary channel proximate the top of the waste storage compartment.

A method as described in claim 6 including receiving waste liquid at a capillary coupled along and open to a length of a waste compartment in a multiple layer cytometric test card, drawing the waste liquid into the capillary via capillary action, transporting the waste via the capillary toward a bottom of the waste compartment, and exiting the waste from the capillary into the waste compartment when surface tension of the waste liquid in the capillary is overcome.

US 2009/0181411A1 discloses a multilayer microfluidic assay device, comprising a waste line coupled to a waste compartment containing an absorbent pad. The waste compartment is in turn coupled to a vent. The absorbent pad in the waste compartment works to assist in promoting directional capillary action and to prevent egress of the waste. The sorbent material also prevents sloshing of the liquid as described in WO 2009/137244A1.

EP 1566215 A2 describes microfluidic systems using vertical grooves to overcome a capillary stop from a narrow channel to a wider compartment.

### Brief Description of the Drawings

FIG. 1 is a block diagram planar representation of a testing card having on board waste storage according to an unclaimed example embodiment.
FIG. 2 is a cross section representation of the testing card of FIG. 1 taken along lines 2—2 according to an example embodiment.
FIG. 3 is an alternative cross section representation of the testing card of FIG. 1 taken along lines 2—2 according to an example embodiment.
FIG. 4 is a block diagram planar representation of an alternative multiple layer testing card having on board waste storage according to the claimed invention.

### Detailed Description

In the following description, reference is made to the accompanying drawings that form a part hereof, and in which is shown by way of illustration specific embodiments which may be practiced. These embodiments are described in sufficient detail to enable those skilled in the art to practice the invention, and it is to be understood that other embodiments may be utilized and that structural, logical and electrical changes may be made without departing from the scope of the present invention. The following description of example embodiments is, therefore, not to be taken in a limited sense, and the scope of the present invention is defined by the appended claims.

Waste storage in a multiple layer cytometric test card can create a backpressure that may interfere with fluid flow upstream of a waste storage chamber. Given a vertical insertion of the card into a test instrument, the waste fluid will settle to the bottom of the waste storage chamber. As waste fluid builds up, the pressure at the bottom of the liquid increases while the pressure at the top stays the same. With prior card designs, waste enters only through the bottom of the waste chamber, resulting in upstream fluid encountering increasing pressure in increasing time as the waste chamber. Flow cytometric measurements may be adversely affected by an increasing backpressure in increasing time. In various embodiments, cytometry is the process of measuring various parameters associated with cells, such as size.

In various embodiments of a new design, waste encounters a waste compartment from the top of the waste compartment, and enters the waste compartment at the top surface of the filling waste liquid, resulting in no change in back pressure as a function of time.

A narrow groove, referred to as a capillary sits along the side of the new waste tank. As the waste encounters the waste compartment, the waste is moved into the capillary via capillary action. The capillary is open to the waste compartment along the length of the waste compartment and is held in the narrow groove by surface tension. Once the surface tension is overcome, either by encountering the bottom of an empty waste compartment or encountering a surface of waste already in the waste compartment, the waste enters the waste compartment. The point at which the waste enters the waste compartment moves up as the waste compartment is filled.

As indicated above, the groove is open to the waste tank. The fluid flows along this groove because of capillary action and is kept in the groove due to surface tension. Very little energy is used to cause the fluid to follow that path, resulting in minimal and unchanging backpressure as the waste compartment fills. If the groove was not present, waste entering the waste compartment from a top waste channel would result in a variable back pressure due to the waste repeatedly overcoming surface tension to intermittently drip into the waste compartment.

Once waste starts to build up along the bottom of the tank, the path of least resistance (as seen by the entering waste fluid) is out onto the surface of the filling waste liquid. This path of least resistance will provide the same negligible backpressure throughout the run.

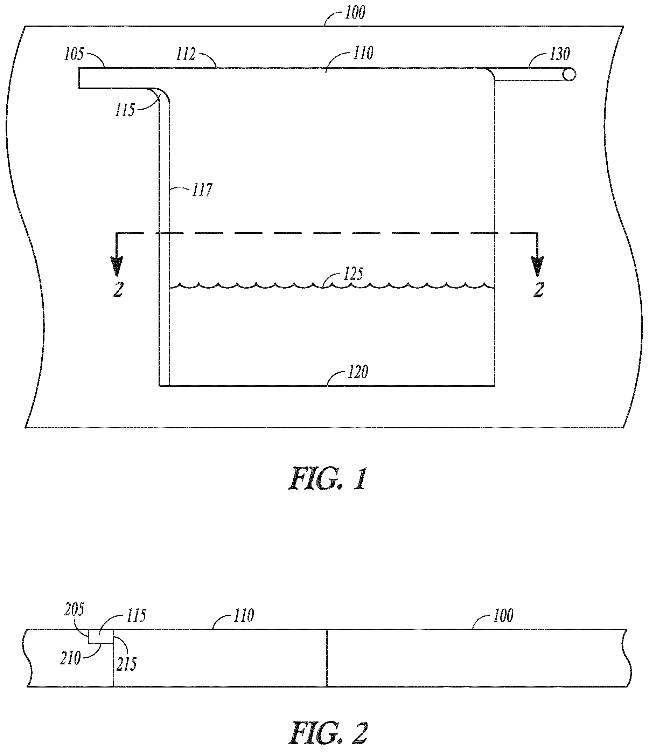

FIG. 1 is a block diagram planar view of a multiple layer test card 100. FIG. 2 is a block diagram cross section of the test card 100 of FIG. 1 taken along line 2—2. In some embodiments, the test card 100 contains many layers of a transparent material such as PET or other acrylic or suitable material that can be patterned with various liquid fluid transport features. The card 100 in some embodiments may be used to perform one or more blood tests utilizing a small volume of blood. The blood or other liquid to be tested, may be transported via one or more layers of the test card, and prepared for analysis by a test instrument into which the card is inserted. Various sensors, such as a combination of light emitting diodes, lasers, and photoreceptors may be used to test the liquid.

After the liquid has been tested, a waste channel 105 receives the waste liquid and transports it to a first waste chamber or compartment 110. The waste channel 105 and waste chamber 110 may be formed in separate layers in some embodiments. In one embodiment, the card 100 is designed to be inserted into the test instrument such that liquid from waste channel 105 enters into a top 112 of the chamber 110.

A narrow groove 115 forms a capillary that the waste liquid enters due to capillary action. The groove 115 extends along a length 117 of the waste chamber 110 to a bottom 120 of the waste chamber, where the liquid overcomes surface tension holding the liquid in the groove 115 when the waste chamber is empty. As the waste chamber 115 fills, as indicated at 125, the waste in the groove 115 enters the waste chamber at the liquid level 125, as the surface tension is overcome by encountering the liquid at the level 125. Air is displaced in the waste chamber 110 and is removed via an air channel 130 where it may be discharged to ambient.

In FIG. 2, the groove 115 is shown in cross section and includes a depth 205 and width 210. In one embodiment, the groove is formed in the same layer as the waste chamber 110. While the waste chamber 110 is cut all the way through the layer and later encased by adjacent layers, the groove may be cut by laser, such as a CO2 laser in a controlled manner to only remove a small portion of the layer. The groove is also encapsulated by at least one adjacent layer. In once embodiment, the depth 205 of the groove 115 is approximately 0.125mm. This depth is sufficient to result in both the capillary action to draw waste liquid into the groove, and is also sufficient to ensure that surface tension of the liquid, indicated at 215 maintains the waste liquid in the groove until the surface tension is overcome by encountering waste liquid already in the waste chamber or encountering the bottom 120 of the waste chamber 115.

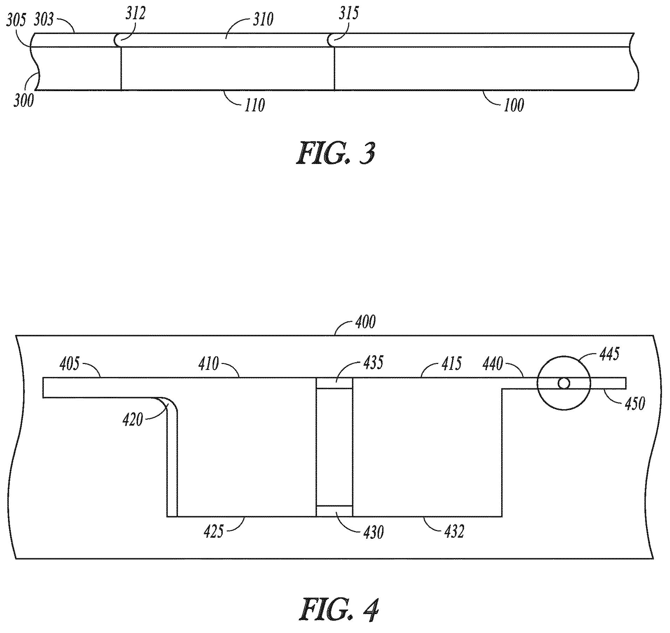

In FIG. 3, the test card 100 includes a layer 300 that includes the waste chamber 110. Test card 100 also includes a second layer 303, coupled to the layer 100 such as via an adhesive layer 305. The second layer 303 includes a cut out portion 310 that helps form a groove 312 that provides the capillary to transport waste into the waste chamber 110. The cut out portion 310 may extend in the second layer 303 toward the other end of the chamber 110 such as shown at 315. In further embodiments, the cut out portion 310 only extends part way toward the end of the chamber 110 to preserve the structural integrity of the second layer

In some embodiments, one or more registration features may be used to ensure the card is properly oriented within the test instrument such that the bottom of the chamber 120 is properly oriented to receive waste liquid from the groove.

In various embodiments, there are multiple layers in one embodiment of the card 100. A layer 210 serves as a cap and also contains the vent passage 150. Layer 212 contains the chambers 110 115, and 120. A layer 213 contains the passes that communicate liquid and air between the chambers and also serves as a cap for one or more other layers 215, 220, 225, and 230 that are transporting and processing the liquid that becomes waste. The number of layers in the card 100 may vary in different embodiments. The vent passage 150 in one embodiment passes air to ambient via layer 230. In further embodiments, vent passage 150 may take an alternate route to pass air to ambient.

FIG. 4 illustrates the card according to the invention 400 having multiple waste chambers. Card 400 includes a waste channel 405 that opens into a first waste chamber 410 that is coupled to a second waste chamber 415. Waste from waste channel 405 is transported by a capillary 420 extending along the length of waste chamber 410 and filling the waste chamber from a bottom 425 of waste chamber 410. A channel 430 couples the first waste chamber 410 to the second waste chamber 415 along the bottoms 425, 432 of both waste chambers. An air pass 435 couples the top portions of the two waste chamber 410 and 415 to transfer air displaced by fluid filling the first chamber 410. A second air pass 440 extends from the top of the second waste chamber 415 to discharge the displaced air from both chambers to ambient. An air permeable membrane 445 may be positioned between the air pass 440 and a pass 450 to ambient, which is formed in a separate layer with the membrane 445 positioned between the two layers to prevent waste liquid from exiting the card 400.

## Claims

1. A multiple layer cytometric test card (400) comprising:
a waste channel (405) to receive biological waste liquid from an area of the test card utilized for testing biological samples;
a first and a second waste storage compartment (410, 415) in a waste layer of the card for storing the waste liquid and having a top and a bottom (425, 432) in relation to an operating vertical orientation of the test card (400);
a narrow groove formed (420) along a vertical length of a side of the first waste storage compartment (410), wherein the waste channel (405) is coupled to the groove (420) at the top of the first waste storage compartment (410), and the groove (420) is open to the first waste storage compartment (410) along the vertical length of the side of the first waste compartment (410) and adapted to transfer waste liquid by capillary action from the waste channel (405) to either the bottom (425) of the first waste storage compartment (410) when the first waste storage compartment (410) is empty or to a surface of waste liquid stored in the first waste storage compartment (410);
a channel (430) coupling the first and second waste storage compartment (410, 415) at bottoms of the first and second waste storage compartment (410, 415);
an air pass (435) coupling the first and second waste storage compartment (410, 415) to transfer air displaced by fluid filling the first waste storage compartment (410) to the second waste storage compartment (415); and
a second air pass (440) coupled to the second waste storage compartment (415) extending from the top of the second waste chamber (415) to discharge the displaced air from both chambers to the ambient, in order to reduce backpressure otherwise resulting from filling the first and second waste storage compartments (410,415).

2. The multiple layer cytometric test card (400) of claim 1 wherein the groove (115) is 0.125 mm in thickness and wherein the waste storage compartment (110) is greater than 0.5 mm in thickness.

3. The multiple layer cytometric test card (400) of claim 1 or claim 2, wherein the groove (115) is formed on a same layer as the waste storage compartments (410, 415).

4. The multiple layer cytometric test card (400) of claim 1 or claim 2, wherein the groove (420) is formed on a capillary layer adjacent a layer containing the waste storage compartments (410, 415), wherein the capillary layer (115) is adapted to cap the waste storage compartments (410,415).

5. The multiple layer cytometric test card (400) of claim 4, wherein the capillary layer and layer containing the waste storage compartments (410, 415) are coupled via an adhesive layer.

6. A method comprising:
receiving waste liquid at a narrow groove (420) formed along a vertical length of a side of a first waste storage compartment (410) in a multiple layer cytometric test card (400);
drawing the waste liquid into the groove (420) via capillary action;
transporting the waste liquid by capillary action via the groove (420) toward a bottom (425) of the first waste storage compartment (425);
exiting the waste liquid from the groove (420) into the first waste storage compartment (410) when the waste liquid encounters either the bottom (425) of the first waste storage compartment (410) when the first waste storage compartment (410) is empty, or a surface of waste liquid stored in the first waste storage compartment (410);
flowing the waste liquid into a second waste storage compartment (415) via a channel (430) coupling the first and second waste storage compartment (410, 415) at bottoms of the first and second waste storage compartment (410, 415);
transferring air displaced by fluid filling the first waste storage compartment (410) to the second waste storage compartment (415) via an air pass (435) coupling the first and second waste storage compartment (410, 415); and
exhausting air from the second waste storage compartment (415) to ambient outside the card (400) to reduce backpressure otherwise resulting from filling the first and second waste storage compartments (410, 415) via a second air pass (440) coupled to the second waste storage compartment (415).

7. The method of claim 6, wherein the air is exhausted through a gas permeable, liquid impermeable membrane (445) separating the second waste storage compartment (415) from ambient.

8. The method of any one of claims 6 to 7, wherein air is passed from the top of the first waste storage compartment (410) to a top of the second waste storage compartment (415).

## Patentansprüche

1. Mehrschichtige zytometrische Prüfkarte (400), umfassend:
einen Abfallkanal (405) zum Aufnehmen von biologischer Abfallflüssigkeit aus einem Bereich der Prüfkarte, die zum Prüfen von biologischen Proben verwendet wird;
eine erste und eine zweite Abfallspeicherkammer (410, 415) in einer Abfallschicht der Karte zum Speichern der Abfallflüssigkeit und aufweisend eine Oberseite und einen Boden (425, 432) bezogen auf eine waagrechte Arbeitsorientierung der Prüfkarte (400);
eine schmale Rinne (420), die entlang einer waagrechten Länge einer Seite der ersten Abfallspeicherkammer (410) gebildet ist, wobei der Abfallkanal (405) an der Oberseite der ersten Abfallspeicherkammer (410) mit der Rinne (420) gekoppelt ist und die Rinne (420) entlang der waagrechten Länge der Seite der ersten Abfallspeicherkammer (410) zu der ersten Abfallspeicherkammer (410) offen ist und dafür ausgelegt ist, Abfallflüssigkeit durch Kapillarwirkung von dem Abfallkanal (405) entweder zu dem Boden (425) der ersten Abfallspeicherkammer (410), wenn die erste Abfallspeicherkammer (410) leer ist, oder zu der Oberfläche von in der ersten Abfallspeicherkammer (410) gespeicherter Abfallflüssigkeit zu überführen;
einen Kanal (430), der die erste und die zweite Abfallspeicherkammer (410, 415) an den Böden der ersten und der zweiten Abfallspeicherkammer (410, 415) koppelt;
einen Luftdurchlass (435), der die erste und die zweite Abfallspeicherkammer (410, 415) koppelt, um Luft, die durch Fluid verdrängt wird, das die erste Abfallspeicherkammer (410) füllt, in die zweite Abfallspeicherkammer (415) zu überführen; und
einen zweiten Luftdurchlass (440), der mit der zweiten Abfallspeicherkammer (415) gekoppelt ist und von der Oberseite der zweiten Abfallspeicherkammer (415) vorragt, um die verdrängte Luft aus beiden Kammern an die Umgebung abzulassen, um Rückdruck zu verringern, der andernfalls durch Füllen der ersten und der zweiten Abfallspeicherkammer (410, 415) entstehen würde.

2. Mehrschichtige zytometrische Prüfkarte (400) gemäß Anspruch 1, wobei die Rinne (115) eine Dicke von 0,125 mm aufweist und wobei die Abfallspeicherkammer (110) eine Dicke von mehr als 0,5 mm aufweist.

3. Mehrschichtige zytometrische Prüfkarte (400) gemäß Anspruch 1 oder Anspruch 2, wobei die Rinne (115) auf der gleichen Schicht wie die Abfallspeicherkammern (410, 415) gebildet ist.

4. Mehrschichtige zytometrische Prüfkarte (400) gemäß Anspruch 1 oder Anspruch 2, wobei die Rinne (420) auf einer Kapillarschicht gebildet ist, die einer Schicht benachbart ist, die die Abfallspeicherkammern (410, 415) enthält, wobei die Kapillarschicht (115) dafür ausgelegt ist, die Abfallspeicherkammern (410, 415) abzudecken.

5. Mehrschichtige zytometrische Prüfkarte (400) gemäß Anspruch 4, wobei die Kapillarschicht und die Schicht, die die Abfallspeicherkammern (410, 415) enthält, über eine Haftschicht gekoppelt sind.

6. Verfahren, umfassend:
Aufnehmen von Abfallflüssigkeit an einer schmalen Rinne (420), die entlang einer waagrechten Länge einer Seite einer ersten Abfallspeicherkammer (410) in einer mehrschichtigen zytometrischen Prüfkarte (400) gebildet ist;
Ziehen der Abfallflüssigkeit durch Kapillarwirkung in die Rinne (420);
Befördern der Abfallflüssigkeit durch Kapillarwirkung durch die Rinne (420) zu dem Boden (425) der ersten Abfallspeicherkammer (425);
Austretenlassen der Abfallflüssigkeit aus der Rinne (420) in die erste Abfallspeicherkammer (410), wenn die Abfallflüssigkeit entweder auf den Boden (425) der ersten Abfallspeicherkammer (410) trifft, wenn die erste Abfallspeicherkammer (410) leer ist, oder auf die Oberfläche von in der ersten Abfallspeicherkammer (410) gespeicherter Abfallflüssigkeit;
Fließenlassen der Abfallflüssigkeit in eine zweite Abfallspeicherkammer (415) durch einen Kanal (430), der die erste und die zweite Abfallspeicherkammer (410, 415) an den Böden der ersten und der zweiten Abfallspeicherkammer (410, 415) koppelt;
Überführen von Luft, die durch Fluid verdrängt wird, das die erste Abfallspeicherkammer (410) füllt, in die zweite Abfallspeicherkammer (415) durch einen Luftdurchlass (435), der die erste und die zweite Abfallspeicherkammer (410, 415) koppelt; und
Ablassen von Luft aus der zweiten Abfallspeicherkammer (415) an die Umgebung außerhalb der Karte (400), um Rückdruck zu verringern, der andernfalls durch Füllen der ersten und der zweiten Abfallspeicherkammer (410, 415) entstehen würde, durch einen zweiten Luftdurchlass (440), der an die zweite Abfallspeicherkammer (415) gekoppelt ist.

7. Verfahren gemäß Anspruch 6, wobei die Luft durch eine gasdurchlässige, flüssigkeitsundurchlässige Membran (445) abgelassen wird, die die zweite Abfallspeicherkammer (415) von der Umgebung trennt.

8. Verfahren gemäß einem der Ansprüche 6 bis 7, wobei Luft von der Oberseite der ersten Abfallspeicherkammer (410) zu der Oberseite der zweiten Abfallspeicherkammer (415) geleitet wird.

## Revendications

1. Carte de test cytométrique à couches multiples (400) comprenant :
un canal de déchets (405) pour recevoir des déchets biologiques liquides depuis une zone de la carte de test utilisée pour analyser des échantillons biologiques ;
un premier et un deuxième compartiment de stockage de déchets (410, 415) dans une couche de déchets de la carte pour stocker les déchets liquides et ayant un sommet et une base (425, 432) par rapport à une orientation verticale opérationnelle de la carte de test (400) ;
une rainure étroite formée (420) le long d'une longueur verticale d'un côté du premier compartiment de stockage de déchets (410), dans laquelle le canal de déchets (405) est couplé à la rainure (420) au sommet du premier compartiment de stockage de déchets (410), et la rainure (420) est ouverte au premier compartiment de stockage de déchets (410) le long de la longueur verticale du côté du premier compartiment de déchets (410) et adaptée pour transférer les déchets liquides par action capillaire depuis le canal de déchets (405) vers la base (425) du premier compartiment de stockage de déchets (410) lorsque le premier compartiment de stockage de déchets (410) est vide ou vers une surface de déchets liquides stockés dans le premier compartiment de stockage de déchets (410) ;
un canal (430) raccordant les premier et deuxième compartiments de stockage de déchets (410, 415) aux bases des premier et deuxième compartiments de stockage de déchets (410, 415) ;
un passage d'air (435) raccordant les premier et deuxième compartiments de stockage de déchets (410, 415) pour transférer l'air déplacé par le fluide remplissant le premier compartiment de stockage de déchets (410) vers le deuxième compartiment de stockage de déchets (415) ; et
un deuxième passage d'air (440) couplé au deuxième compartiment de stockage de déchets (415) s'étendant depuis le sommet de la deuxième chambre de déchets (415) pour décharger l'air déplacé depuis les deux chambres vers l'air ambiant, afin de réduire la contre-pression résultant autrement du remplissage des premier et deuxième compartiments de stockage de déchets (410, 415).

2. Carte de test cytométrique à couches multiples (400) de la revendication 1 dans laquelle la rainure (115) est de 0,125 mm d'épaisseur et dans laquelle le compartiment de stockage de déchets (110) a une épaisseur supérieure à 0,5 mm.

3. Carte de test cytométrique à couches multiples (400) de la revendication 1 ou la revendication 2, dans laquelle la rainure (115) est formée sur une même couche que les compartiments de stockage de déchets (410, 415).

4. Carte de test cytométrique à couches multiples (400) de la revendication 1 ou la revendication 2, dans laquelle la rainure (420) est formée sur une couche capillaire adjacente à une couche contenant les compartiments de stockage de déchets (410, 415), dans laquelle la couche capillaire (115) est adaptée pour recouvrir les compartiments de stockage de déchets (410, 415).

5. Carte de test cytométrique à couches multiples (400) de la revendication 4, dans laquelle la couche capillaire et la couche contenant les compartiments de stockage de déchets (410, 415) sont couplées par l'intermédiaire d'une couche adhésive.

6. Procédé comprenant :
la réception de déchets liquides au niveau d'une rainure étroite (420) formée le long d'une longueur verticale d'un côté d'un premier compartiment de stockage de déchets (410) dans une carte de test cytométrique à couches multiples (400) ;
l'aspiration des déchets liquides dans la rainure (420) par action capillaire ;
le transport des déchets liquides par action capillaire via la rainure (420) vers une base (425) du premier compartiment de stockage de déchets (425) ;
la sortie des déchets liquides de la rainure (420) dans le premier compartiment de stockage de déchets (410) lorsque les déchets liquides atteignent la base (425) du premier compartiment de stockage de déchets (410) lorsque le premier compartiment de stockage de déchets (410) est vide, ou une surface de déchets liquides stockés dans le premier compartiment de stockage de déchets (410) ;
l'écoulement des déchets liquides dans un deuxième compartiment de stockage de déchets (415) via un canal (430) raccordant les premier et deuxième compartiments de stockage de déchets (410, 415) aux bases des premier et deuxième compartiments de stockage de déchets (410, 415) ;
le transfert de l'air déplacé par remplissage de fluide du premier compartiment de stockage de déchets (410) vers le deuxième compartiment de stockage de déchets (415) via un passage d'air (435) raccordant les premier et deuxième compartiments de stockage de déchets (410, 415) ; et
l'échappement de l'air depuis le deuxième compartiment de stockage de déchets (415) vers l'air ambiant à l'extérieur de la carte (400) afin de réduire la contre-pression résultant autrement du remplissage des premier et deuxième compartiments de stockage de déchets (410, 415) via un deuxième passage d'air (440) couplé au deuxième compartiment de stockage de déchets (415).

7. Procédé de la revendication 6, dans lequel l'air est échappé à travers une membrane perméable aux gaz, imperméable aux liquides (445) séparant le deuxième compartiment de stockage de déchets (415) de l'air ambiant.

8. Procédé de l'une quelconque des revendications 6 à 7, dans lequel l'air est amené à passer du sommet du premier compartiment de stockage de déchets (410) à un sommet du deuxième compartiment de stockage de déchets (415).
